# EUROPÄISCHE PATENTSCHRIFT

(11) **EP 3 287 805 B1**
(45) Veröffentlichungstag und Bekanntmachung des Hinweises auf die Patenterteilung: **26.06.2019**
(21) Anmeldenummer: 17170596.5
(22) Anmeldetag: 11.05.2017
(51) Int. Cl.: G01R 33/36, G01R 33/28, G01R 33/34

(54) **SAR SICHERHEITS-VORRICHTUNG UND -VERFAHREN ZUM ERKENNEN VON ENT-VERSTIMMUNG VON MR ANTENNENSPULEN.**
SAR SAFETY DEVICE AND METHOD FOR DETECTING UN-DETUNED MRI ANTENNA COILS.
DISPOSITIF ET PROCÉDÉ DE SÉCURITÉ DAS PAR RECONNAISSANCE DE NON DÉSACCORDAGE DE BOBINES D'ANTENNE IRM.

(43) Veröffentlichungstag der Anmeldung: 28.02.2018
(73) Patentinhaber: Siemens Healthcare GmbH, 91052 Erlangen (DE)
(72) Erfinder: Nistler, Jürgen, 91056 Erlangen (DE); Vester, Markus, 90471 Nürnberg (DE); Wünsch, Christian, 90552 Röthenbach a.d.Pegnitz (DE)

(56) Entgegenhaltungen:
- WO-A2-2015/091544
- DE-A1-102006 061 740
- US-A1- 2010 244 840
- US-A1- 2016 097 829

## Beschreibung

Die Erfindung betrifft eine Vorrichtung sowie ein Verfahren zum Erkennen einer Antennenspule mit nicht aktiver Verstimmungsvorrichtung sowie einen Magnetresonanztomographen mit einer derartigen Vorrichtung. Die Vorrichtung weist einen Sender, eine Antenne, einen Amplitudenmesser und eine Steuerung auf. Die Steuerung ist ausgelegt, den Sender derart anzusteuern, dass er Hochfrequenzsignale mit unterschiedlichen, vorbestimmten Amplitude über die Antenne aussendet und mit dem Amplitudenmesser Prüfamplituden in Abhängigkeit von dem ausgesendeten Signal erfasst.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Antennen, sogenannte Lokalspulen verwendet, die zur Erzielung eines besseren Signal-Rauschabstandes unmittelbar am Untersuchungsobjekt angeordnet werden.

Die Lokalspulen sind für den Empfang auf der Larmorfrequenz, vorzugsweise der Larmorfrequenz eines Wasserstoffkerns im statischen Magnetfeld B0 des Magnetresonanztomographen, resonant eingestellt. Während des Anregungspulses für die Kernspins mit der gleichen Frequenz müssen sie jedoch verstimmt werden, um eine Zerstörung aufgrund hoher induzierter Spannungen bzw. Ströme zu vermeiden. Die Verstimmung erfolgt zum einen durch aktive Bauelemente wie PIN-Dioden, die durch einen Steuerstrom leitfähig gemacht werden. Ist die Lokalspule nicht angeschlossen, sind passive Schutzelemente wie gekreuzte Dioden oder Schmelzsicherungen vorgesehen. Sind diese Schutzmechanismen jedoch defekt, kann es aufgrund der Resonanzbedingungen der Lokalspule in deren Umgebung zu überhöhten Feldstärken während des Anregungspulses kommen, die trotz einer generellen Einhaltung globaler Grenzwerte eine zu untersuchende Person potentiell gefährden. Dies ist insbesondere der Fall, wenn z.B. wegen Implantaten eine besondere Gefährdungslage vorliegt und bereits eine kurzzeitige Überschreitung von Feldstärke-Grenzwerten den Patienten gefährdet.

Aus der Druckschrift DE 10 2015 217 723 A1 ist beispielsweise eine lokale Sendespule mit einer Überwachungseinrichtung bekannt.

Das Dokument US 2016/0097829 A1 beschreibt eine Lokalspulenmatrix, die mit einer einzigen Übertragungsleitung angeschlossen ist. Die Lokalspulenmatrix weist ein aktives Element aus, das über die koaxiale Übertragungsleitung versorgt wird. Die Lokalspulenmatrix weist weiterhin eine Verstimmeinrichtung auf. Die Spannung auf der Übertragungsleitung wechselt zwischen einer ersten und einer zweiten Gleichspannung gleicher Polarität, wodurch die Verstimmeinrichtung die Lokalspulenmatrix verstimmt oder die Verstimmeinrichtung deaktiviert.

Es ist daher eine Aufgabe der vorliegenden Erdfindung, Untersuchungen mittels eines Magnetresonanztomographen insbesondere für Patienten mit Implantaten sicherer zu machen.

Die Aufgabe wird durch eine erfindungsgemäße Vorrichtung nach Anspruch 1 sowie einen Magnetresonanztomographen nach Anspruch 7 und ein Verfahren nach Anspruch 8 gelöst.

Die erfindungsgemäße Vorrichtung zum Erkennen einer Antennenspule mit nicht aktiver Verstimmungsvorrichtung weist einen Sender, eine Antenne, einen Amplitudenmesser und eine Steuerung auf. Vorzugsweise ist der Sender ausgelegt, Hochfrequenz mit der Frequenz einer Larmorfrequenz auszusenden, auf der die Antennenspule ausgelegt ist, ein Magnetresonanzsignal zu empfangen. Die Antennenspule kann beispielsweise eine Lokalspule oder eine Körperspule sein oder auch ein Element davon.

Dabei ist die Steuerung ausgelegt, den Sender derart anzusteuern, dass er Hochfrequenzsignale mit unterschiedlichen vorbestimmten Amplituden über die Antenne aussendet. Vorzugsweise handelt es sich dabei um Amplituden, die bei einer resonanten Antennenspule in einem Wirkungsbereich der Antenne eine Spannung beispielsweise in einem Bereich zwischen 0,1V und 2V, 0,2V und 1V oder 0,4V und 0,8V erzeugt. Vorzugsweise handelt es sich bei der induzierten Spannung um eine Spannung, die in einem Bereich einer Nicht-Linearität einer Kennlinie eines Schutzelementes wie beispielsweise einer Diode, Zenerdiode oder PIN-Diode liegt.

Die Steuerung steht mit einem Amplitudenmesser in Signalverbindung und ist weiterhin ausgelegt, mit dem Amplitudenmesser Prüfamplituden in Abhängigkeit von dem ausgesendeten Signal zu erfassen. Bei den Prüfamplituden kann es sich beispielsweise um Amplituden handeln, die proportional zu einer magnetischen Feldkomponente der Hochfrequenzsignale sind, wie sie durch eine Aufnahme-Spule (Pick-up-coil) erfasst werden kann. Denkbar ist auch eine Amplitude in Abhängigkeit von einer elektrischen Feldstärke, die beispielsweise durch einen Dipol erfasst werden kann. Ebenfalls sind auch Amplituden von Spannungen und/oder Strömen in Zuleitungen der Antenne möglich. Schließlich sind auch Amplituden von Signalen denkbar, die nicht linear von Strom oder Spannung abhängen, wie beispielsweise die Leistung mit quadratischer Abhängigkeit. Schließlich ist es auch denkbar, dass über die Amplitude andere Größen wie eine Phase von der Steuerung erfasst werden. Dabei ist erfindungsgemäß "in Abhängigkeit von dem ausgesendeten Signal" dahingehend zu verstehen, dass die Prüfamplituden in Echtzeit oder nahezu in Echtzeit durch in der Hochfrequenztechnik beim Empfang übliche Schritte wie z.B. Verstärken, Abschwächen, Filtern, Mischen oder Gleichrichten aus einem von dem ausgesendeten Signal z.B. durch Induktion, erzeugten Eingangssignal abgeleitet werden. Vorzugsweise ist die Steuerung ausgelegt, mindestens zwei Prüfamplituden in Abhängigkeit von zwei unterschiedlichen vorbestimmten Amplituden zu erfassen. Weiterhin ist die Steuerung ausgelegt, eine Prüfrelation zwischen den vorbestimmten Amplituden und den erfassten Prüfamplituden zu bestimmen. Denkbar ist es beispielsweise, dass die Steuerung eine Differenz der Werte oder einen Quotienten aus vorbestimmter Amplitude und erfasster Prüfamplitude bildet. Ebenfalls ist es möglich, dass die Steuerung die Werte vorher oder nachher mit zusätzlichen Schritten bearbeitet, beispielsweise eine Potenzfunktion oder einen Logarithmus darauf anwendet.

Schließlich ist die Steuerung ausgelegt, die gebildete Prüfrelation mit einer vorbestimmten Referenzrelation zu vergleichen. Beispielsweise kann die Steuerung überprüfen, ob die Feldstärke linear mit der an der Antenne angelegten Spannung steigt. Es ist dann denkbar, dass eine Antennenspule mit Schutzdiode oberhalb einer gewissen Feldstärke in einen leitenden Bereich der Kennlinie der Schutzdiode kommt und wegen der von der Antennenspule aufgenommenen Leistung kein linearer Zusammenhang mehr zwischen Spannung an der Antenne und der Feldstärke mehr besteht.

Die Steuerung ist dabei ausgelegt, beim Erkennen einer entsprechenden Abweichung der bestimmten Prüfrelation von einer vorbestimmten Referenzrelation ein Signal auszugeben.

Die vorbestimmte Referenzrelation ist eine lineare Relation. Die vorbestimmte Relation ist vorzugsweise in einem Speicher der Steuerung gespeichert, beispielsweise als Parametersatz mit Koeffizienten oder Wertetabelle. Als lineare Relation ist dabei zu verstehen, dass die Abhängigkeit zwischen vorbestimmter Amplitude und Prüfamplitude einer linearen Gleichung der Form y = ax + b folgt bzw. davon lediglich im Rahmen von Messungenauigkeiten um höchstens 1%, 5%, 10% oder 20% abweicht. Darunter ist auch der Sonderfall einer linearen Relation mit einem konstanten Wert zu verstehen, der aber von dem Wert Null verschieden ist.

Elektrische und magnetische Felder folgen linearen Gesetzmäßigkeiten, sofern nicht unlineare Elemente wie beispielsweise eine Verstimmung durch Dioden vorliegen. Durch Vergleich mit einer linearen Kennlinie und Abweichungen davon kann so auf vorteilhafte Weise eine entsprechende Sicherungstechnik und deren ordnungsgemäße Funktion geschlossen werden.

Auf vorteilhafte Weise ermöglicht die erfindungsgemäße Vorrichtung, eine Antennenspule mit nicht aktivierter Verstimmung zu erkennen, bevor durch eine hohe Hochfrequenzleistung ein möglicher Schaden an der Antennenspule auftritt oder ein Patient gefährdet wird.

Der erfindungsgemäße Magnetresonanztomograph und das erfindungsgemäße Verfahren teilen die Vorteile der erfindungsgemäßen Vorrichtung.

Weitere vorteilhafte Ausführungsformen sind in den Unteransprüchen angegeben.
In einer denkbaren Ausführungsform der erfindungsgemäßen Vorrichtung weist der Amplitudenmesser einen Richtkoppler auf. Der Richtkoppler ist vorzugsweise zwischen dem Sender und der Antenne angeordnet, um eine von der Antenne reflektierte Leistung zu erfassen, die sich beispielsweise durch eine veränderte Impedanz der Antenne durch einen Resonator im Untersuchungsbereich ändert. Dabei weisen die erfassten Prüfamplituden in dieser Ausführungsform eine Abhängigkeit von einer reflektierten Leistung auf.

Ein Richtkoppler bietet auf vorteilhafte Weise eine einfache Möglichkeit, mit der unerwünscht zurücklaufenden Hochfrequenzenergie ohne Rückgriff auf die vorlaufende nützliche Sendeleistung eine Information über den Untersuchungsbereich bezüglich Hochfrequenzstrahlung zu erfassen.

In einer denkbaren Ausführungsform der erfindungsgemäßen Vorrichtung ist die Steuerung ausgelegt, die Prüfrelation in Abhängigkeit von einer Phase der von dem Amplitudenmesser erfassten Prüfamplitude zu bestimmen. So ist es beispielsweise denkbar, dass die vorbestimmte Amplitude eine Spannung einer Senderendstufe ist, die durch ein Treibersignal von der Steuerung vorgegeben ist. Als Prüfamplitude könnte dann von dem Amplitudenmesser auch ein Strom zwischen Senderendstufe und Antenne erfasst werden. Für Impedanzen an der Antenne mit ohmschen Verhalten bleibt dabei die Phase bzw. Phasenbeziehung zwischen Spannung und Strom konstant, während eine Belastung durch einen komplexen Widerstand, wie einem resonanten Schwingkreis, eine Phasenverschiebung verursacht. Ist dabei der komplexe Widerstand selbst auf unlineare Weise von der Sendeleistung abhängig, wie es beispielsweise bei einer Diode im Resonanzkreis der Fall ist, ergibt sich auch für die Phase eine nichtlineare Relation.

Auf vorteilhafte Weise kann über die Phase mit hoher Empfindlichkeit eine Schutzvorrichtung einer Antennenspule erkannt werden.

In einer möglichen Ausführungsform der erfindungsgemäßen Vorrichtung ist die Steuerung ausgelegt, die Prüfrelation in Abhängigkeit von einer über die Antenne ausgesendeten Leistung zu bestimmen. Dabei ist es beispielsweise denkbar, dass der Amplitudenmesser ausgelegt ist, Strom und Spannung zu erfassen und über das Produkt aus Spannung und Strom die Leistung zu ermitteln. Es wäre aber auch denkbar, aus einem der beiden Werte unter Annahme einer im Wesentlichen konstanten Impedanz eine Leistung zu errechnen. Dies ist insbesondere denkbar, wenn die Änderungen an dem Messwert gering, d.h. z.B. kleiner als 5% oder 10% sind. Dies ist zum Beispiel möglich, wenn die Antenne im Wesentlichen an die Impedanz der Zuleitung angepasst ist und die Prüfamplitude eine reflektierte Leistung ist, die nur dann auftritt, wenn sich die Anpassung ändert. Schließlich ist es grundsätzlich auch möglich die Leistung direkt, zum Beispiel bolometrisch zu erfassen.

Die Leistung ist eine Größe, die unabhängig von einer möglicherweise ortsabhängigen Phasenlage zwischen Strom und Spannung auf vorteilhafte Weise eine Leistungssenke in Form einer Antennenspule erfassen kann.

In einer denkbaren Ausführungsform der erfindungsgemäßenVorrichtung ist die Vorrichtung, vorzugsweise die Steuerung, ausgelegt, in Abhängigkeit von dem ausgegebenen Signal eine Aussendung von Hochfrequenz durch den Sender zu unterbrechen.

Auf vorteilhafte Weise kann die Vorrichtung eine Aussendung von Hochfrequenzstrahlung durch den Sender unterbrechen, bevor ein Schaden auftritt, beispielsweise wenn eine nicht angeschlossene Antennenspule in einem Untersuchungsbereich angeordnet ist.

In einer möglichen Ausführungsform der erfindungsgemäßen Vorrichtung ist diese ausgelegt, über die Antenne eine Mehrzahl unterschiedlicher vorbestimmter Moden auszusenden. Denkbar ist als Antenne beispielsweise ein Array mit mehreren unabhängigen Sendeelementen, die von einem Sender über eine Verteilmatrix unterschiedlich ansteuerbar sind. Möglich sind auch mehrere unabhängige Sendemodule in einem Sender. Ein derartiges Sendearray kann in einer Körperspule mit mehreren Sendeelementen oder in einer Lokalspule mit mehreren Spulenelementen vorgesehen sein. Da die unterschiedlichen Moden unterschiedliche Polarisation aufweisen, ist es dabei denkbar, dass einzelne Moden eine Antennenspule bei einer entsprechenden Ausrichtung der Antennenspule nicht oder nur in geringem Maß anregen. Die erfindungsgemäße Vorrichtung ist deshalb ausgelegt, in dieser Ausführungsform mittels einer Mehrzahl an Amplitudenmessern eine Mehrzahl an Prüfamplituden zu erfassen, und in Abhängigkeit von den erfassten Prüfamplituden eine oder mehrere Prüfrelationen zu bestimmen. Als Mehrzahl an Amplitudenmessern ist auch ein einzelner Amplitudenmesser mit einem Multiplexer mit einer Vielzahl an Eingängen für die zu erfassenden Eingänge zu verstehen.

Auf vorteilhafte Weise ist die erfindungsgemäße Vorrichtung auch in der Lage, Prüfamplituden für unterschiedliche Moden zu erfassen und so auch Antennenspulen in unterschiedlichen Orientierungen zur Antenne zu erkennen.

In einer denkbaren Ausführungsform des erfindungsgemäßen Magnetresonanztomographen ist der Sender ausgelegt, einen Anregungspuls für Kernspins zu erzeugen. Insbesondere weist der Sender die erforderliche Sendeleistung von beispielsweise mehr als einem Kilowatt auf, um Kernspins in ausreichendem Maß anzuregen.

Dadurch ist die Vorrichtung in der Lage, mittels der vorhandenen Infrastruktur des Magnetresonanztomographen aus Sender und Antenne, z.B. der Körperspule, nicht angeschlossene Antennenspulen, z.B. in Form von Lokalspulen, zu erkennen.

In einer möglichen Ausführungsform des erfindungsgemäßen Verfahrens weist dieses den Schritt auf, eine Verstimmungsvorrichtung einer Antennenspule zu deaktivieren. Beispielsweise kann die Steuerung eine Stromzufuhr zu einer PIN-Diode oder einem anderen Schaltelement in der Antennenspule unterbrechen, sodass diese den Resonanzkreis nicht mehr kurzschließt. Dabei befindet sich die Antennenspule in einem Bereich, der bei einer Aktivierung des Senders eine relevante Feldstärke der Hochfrequenzsignale aufweist. Als relevante Feldstärke wird dabei insbesondere eine Feldstärke des Hochfrequenzfeldes angesehen, die in dem ungedämpften Resonanzkreis der Antennenspule eine Spannung von mehr als 0,5 V oder 1 V erzeugt.

Auf vorteilhafte Weise ist es mit der erfindungsgemäßen Vorrichtung in dem Verfahren auch möglich, nicht nur nicht angeschlossene Antennenspulen zu erkennen, sondern auch die Funktion einer Verstimmungseinrichtung bei einer angeschlossenen Antennenspule zu überprüfen. Dies gilt insbesondere für passive zusätzliche Schutzeinrichtungen wie Sicherungen oder gekreuzte Dioden, die ohne Ansteuerung von außen wirksam sind.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanztomographen mit einer beispielhaften erfindungsgemäßen Vorrichtung;
- Fig. 2: eine schematische Darstellung einer möglichen Ausführungsform einer erfindungsgemäßen Vorrichtung;
- Fig. 3: eine schematische Darstellung einer möglichen Ausführungsform einer erfindungsgemäßen Vorrichtung;
- Fig. 4: ein schematisches Ablaufschema für ein beispielhaftes erfindungsgemäßes Verfahren.

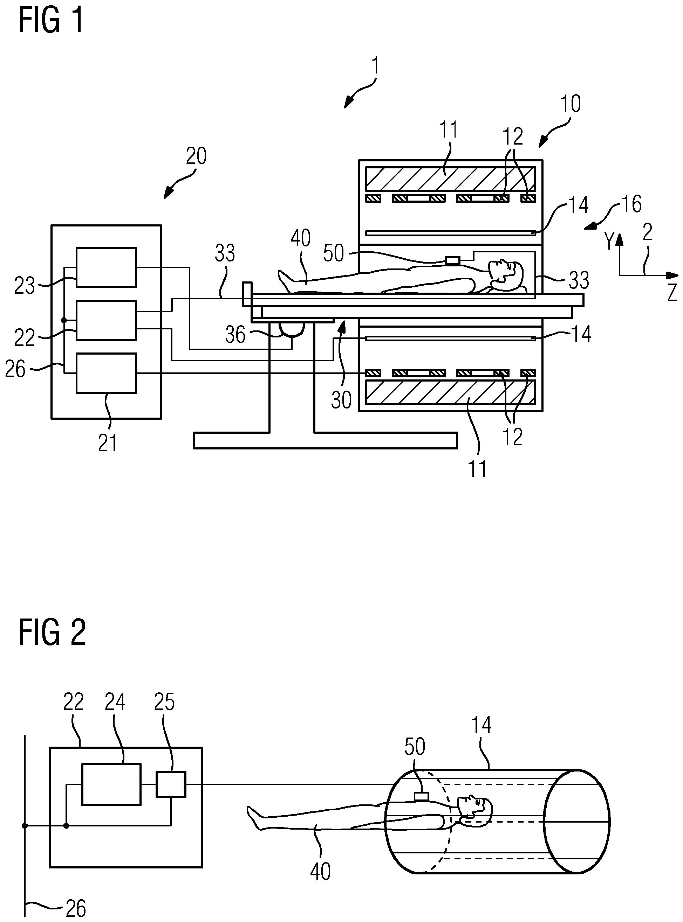

Fig. 1 zeigt eine schematische Darstellung einer Ausführungsform eines erfindungsgemäßen Magnetresonanztomographen 1.

Die Magneteinheit 10 weist einen Feldmagneten 11 auf, der ein statisches Magnetfeld B0 zur Ausrichtung von Kernspins von Proben bzw. Patienten 40 in einem Aufnahmebereich erzeugt. Der Aufnahmebereich ist in einem Patiententunnel 16 angeordnet, der sich in einer Längsrichtung 2 durch die Magneteinheit 10 erstreckt. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 40 emittierte Resonanzsignale zu empfangen und über eine Signalleitung abzugeben. Bevorzugter Weise wird aber die Körperspule 14 für das Empfangen des Hochfrequenzsignals durch Lokalspulen 50 ersetzt, die in dem Patiententunnel 16 nahe am Patient 40 angeordnet sind. Es ist aber auch denkbar, dass die Lokalspule 50 zum Senden und Empfangen ausgelegt ist.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.
Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 40 zu erzeugen. Dabei können Pulsleistungen im Bereich von Kilowatt erreicht werden.

In Fig. 2 ist eine beispielhafte Ausführungsform der erfindungsgemäßen Vorrichtung dargestellt. In Fig. 2 sind gleiche Gegenstände mit gleichen Referenzzeichen wie in Fig. 1 bezeichnet. Aus Gründen der Übersichtlichkeit sind dabei nicht alle Gegenstände aus Fig. 1 dargestellt.

In der Hochfrequenzeinheit 22 ist ein Sender 24 zur Erzeugung von Anregungspulsen mit der Larmorfrequenz des Magnetresonanztomographen vorgesehen. Vorzugsweise handelt es sich dabei um die Larmorfrequenz von Wasserstoffkernen im Magnetfeld B0 des Feldmagneten 11, es sind aber auch Kerne anderer Elemente denkbar. Der Sender 24 wird dabei von der Steuerung 23 des Magnetresonanztomographen 1 über den Signalbus 26 gesteuert, sodass er Anregungspulse mit vorbestimmter Frequenz, Phase, spektraler Verteilung und/oder Leistung erzeugen kann, die über eine Signalverbindung zu der Körperspule 14 als Antenne übertragen werden. Dabei sind die Steuerung 23 und der Sender 24 ausgelegt, die Leistung in einem weiten Bereich, der von wenigen Watt bis einigen Kilowatt reichen kann, einzustellen. Insbesondere kann die Leistung des Senders 24 soweit reduziert werden, dass der Patient und auch eine nicht angeschlossene Antennenspule, hier die Lokalspule 50, nicht gefährdet bzw. zerstört werden.

Zwischen Sender 24 und Körperspule 14 ist ein Amplitudenmesser 25 angeordnet, der einen Messwert des Ausgangssignals des Senders 24 erfassen kann. Dies sind beispielsweise Spannung oder/und Strom. Es ist auch denkbar, dass der Amplitudenmesser eine Leistung erfasst, indem Strom und Spannung erfasst und multipliziert werden, oder indem direkt die Leistung mittels ihrer Wirkung, z.B. bolometrisch erfasst wird.

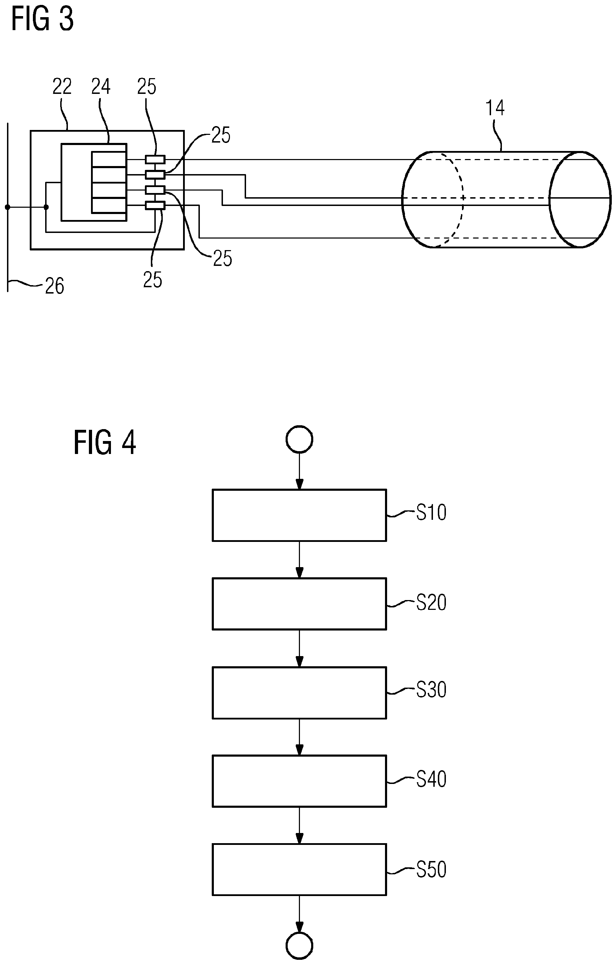

In Fig. 3 ist eine Variante der erfindungsgemäßen Vorrichtung dargestellt, bei der der Sender 24 eine Mehrzahl an Sendemodulen aufweist, die voneinander unabhängig Hochfrequenzsignale erzeugen können, sodass beispielsweise mit einer Körperspule 14 mit unabhängigen Strahlerelementen unterschiedliche Moden mit unterschiedlicher räumlicher Verteilung und Polarisation ausgebildet werden können. Die unterschiedlichen Moden erlauben es auf diese Weise, auch eine Lokalspule 50 anzuregen, deren Spulenwicklung möglicherweise orthogonal zu einer ersten Mode ausgerichtet ist und daher mit dieser nicht angeregt werden könnte. Mehrere Amplitudenmesser 25 erfassen dabei jeweils eine Prüfamplitude eines Sendermoduls. Es ist aber auch denkbar, dass nur ein Amplitudenmesser 25 vorgesehen ist, der über einen Multiplexer die Prüfamplituden unterschiedlicher Sendermodule zugeführt bekommt.

Die Amplitudenmesser 25 können beispielsweise auch Richtkoppler aufweisen, die das Erfassen einer von der Antenne reflektierten Leistung ermöglichen.

In Fig. 4 ist ein schematischer Ablaufplan einer möglichen Ausführungsform des erfindungsgemäßen Verfahrens dargestellt.

In einem Schritt S20 gibt die Steuerung 23 über den Signalbus 26 dem Sender 24 die Anweisung, Hochfrequenzsignale mit unterschiedlicher vorbestimmter Amplitude über die Antenne, beispielsweise die Körperspule 14 auszusenden. Dabei sind die Amplituden so gewählt, dass eine Gefährdung der Antennenspulen, beispielsweise der Lokalspule 50 und insbesondere des Patienten ausgeschlossen ist. Beispielsweise ist die Leistung kleiner als 5, 10 oder 100 Watt, sodass eine in einer resonanten, ungedämpften Antennenspule induzierte Spannung kleiner als 1 V, 2 V oder 5 V ist.
In einem Schritt S30 erfasst der Amplitudenmesser 25 Prüfamplituden in Abhängigkeit von dem ausgesendeten Signal. In der Ausführungsform der Fig. 3 kann dies beispielsweise eine Spannung auf der zu der Antenne führenden Signalleitung sein. Denkbar ist es aber auch, dass Spannung und Strom oder dass mittels Richtkoppler eine von der Antenne reflektierte und/oder zu der Antenne fließende Leistung von dem Amplitudenmesser erfasst werden. Der Amplitudenmesser kann dabei auch weiter durch digitale oder analoge Signalbearbeitung wie beispielsweise Gleichrichtung oder Filterung aufbereitete Signale erfassen, die von dem von dem Sender 24 ausgesendeten Signal abhängen und in Bezug zu dessen Leistung oder auch Phase stehen. Der wesentliche Punkt für die erfassten Signale ist es, dass diese einen Rückschluss auf Hochfrequenzeigenschaften im Patiententunnel 16 erlauben bzw. davon beeinflusst sind.

Die Schritte S20 und S30 werden dabei vorzugsweise mit unterschiedlichen Amplituden wiederholt. Beispielsweise kann mit einer kleinen vorbestimmten Amplitude der zu sendenden Signale begonnen werden und diese in Schritten, beispielsweise mit konstantem Abstand oder auch exponentiell erhöht werden. Auf diese Weise kann mit sicheren kleinen Feldstärken begonnen werden. Um nachfolgend eine Unterscheidung zwischen einem linearen Anstieg des Prüfsignals von einem nichtlinearen zu unterscheiden, sind mindestens drei Wiederholungen mit unterschiedlichen vorbestimmten Amplituden erforderlich, wobei eine mögliche Messung mit einer vorbestimmten Amplitude Null entfallen kann.

In einem Schritt S40 bestimmt vorzugsweise die Steuerung 23 eine Prüfrelation zwischen den vorbestimmten Amplituden und den erfassten Prüfamplituden. Es ist aber auch denkbar, dass eine dedizierte analoge oder digitale
Signalverarbeitungseinheit diesen Schritt ausführt. Im einfachsten Fall ist es denkbar, dass die Steuerung 23 dazu jeweils einen Quotienten aus der jeweils vorbestimmter Amplitude und der dazugehörigen Prüfamplitude bildet. Der Quotient gibt dann eine Steigung einer linearen Relation an. Aufgrund der Maxwellschen Gesetze sind die zu erwartenden Zusammenhänge zwischen den elektrischen und magnetischen Größen zunächst linear. Erst wenn nichtlineare Elemente wie beispielsweise Schutzdioden mit den elektromagnetischen Wechselfeldern verkoppelt werden und die induzierten Spannungen in einem nichtlinearen Bereich der Kennlinien dieser Elemente liegen, weicht dieser Zusammenhang von einer linearen Relation ab.

In einem Schritt S50 gibt beispielsweise die Steuerung 23 ein Signal aus, wenn sie eine Abweichung der bestimmten Prüfrelation von einer vorbestimmten Referenzrelation feststellt. Die Referenzrelation ist, im einfachsten Fall eine lineare Relation, die den zu erwartenden linearen Zusammenhang zwischen der vorbestimmten ausgegebenen Amplitude und der Prüfamplitude wiederspiegelt. Es kann aber auch sein, dass in der Referenzrelation bereits Nichtlinearitäten des Amplitudenmessers 25 berücksichtigt sind und diese deshalb zum Beispiel zumindest Abschnittsweise einem Potenz- oder Exponentialverlauf folgt. Dies kann beispielsweise das Verhalten einer Gleichrichterdiode im Amplitudenmesser 25 bedingt sein. Erst wenn die bestimmte Relation von diesem erwarteten Verhalten abweicht, wird das Signal erzeugt. Vorzugsweise dient das Signal dazu, eine weitere Aussendung von Hochfrequenzleistung durch den Sender 24 zu unterbrechen oder zumindest zu reduzieren, denn es besteht in diesem Fall der Verdacht, dass sich eine nicht verstimmte Antennenspule in dem Patiententunnel befindet.

In einem denkbaren Schritt S10 einer Ausführungsform des erfindungsgemäßen Verfahrens deaktiviert die Steuerung 23 eine Verstimmungseinrichtung einer Antennenspule, beispielsweise der Lokalspule 50, indem eine Versorgungsspannung für eine PIN-Diode unterbrochen wird, die in der Lokalspule 50 einen Resonanzkreis mit einer Spulenwicklung der Lokalspule 50 kurschließt. Die Lokalspule 50 ist dann mit der von dem Sender 24 über die Körperspule 14 ausgestrahlten Hochfrequenz resonant verkoppelt und entnimmt dem Hochfrequenzfeld Leistung. Passive Schutzmechanismen wie beispielsweise gekreuzte Dioden in der Lokalspule 50 verhindern dabei mit zunehmender Leistung des Hochfrequenzfeldes durch ihre nichtlineare Kennlinie das Entstehen zu hoher Spannungen in der Lokalspule 50, entnehmen dabei aber auch exponentiell zunehmend Leistung aus dem Hochfrequenzfeld und führen somit im einfachsten Fall über eine nichtlineare Relation zwischen vorbestimmter Amplitude und Prüfamplitude zu der Erkenntnis, dass eine Antennenspule vorhanden ist und dass der passive Schutzmechanismus einwandfrei funktioniert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Ansprüche zu verlassen.

## Patentansprüche

1. Vorrichtung zum Erkennen einer Antennenspule mit nicht aktiver Verstimmungsvorrichtung, wobei die Vorrichtung einen Sender (24), eine Antenne, einen Amplitudenmesser (25) und eine Steuerung (23) aufweist,
wobei die Steuerung (23) ausgelegt ist, den Sender (24) derart anzusteuern, dass er Hochfrequenzsignale mit unterschiedlichen vorbestimmten Amplitude über die Antenne aussendet, mit dem Amplitudenmesser (25) Prüfamplituden in Abhängigkeit von dem ausgesendeten Signal zu erfassen, eine Prüfrelation zwischen den vorbestimmten Amplituden und den erfassten Prüfamplituden zu bestimmen und bei Abweichung der bestimmten Prüfrelation von einer vorbestimmten Referenzrelation ein Signal auszugeben, wobei die vorbestimmte Referenzrelation in der Steuerung (23) eine lineare Relation ist.

2. Vorrichtung nach Anspruch 1, wobei der Amplitudenmesser (25) einen Richtkoppler aufweist und ausgelegt ist, die Prüfamplituden in Abhängigkeit von einer reflektierten Leistung zu erfassen.

3. Vorrichtung nach einem der vorhergehenden Ansprüche, wobei die Steuerung (23) ausgelegt ist, die Prüfrelation in Abhängigkeit von einer Phase der von dem Amplitudenmesser (25) erfassten Prüfamplitude zu bestimmen.

4. Vorrichtung nach einem der vorhergehenden Ansprüche, wobei die Steuerung (23) ausgelegt ist, die Prüfrelation in Abhängigkeit von einer über die Antenne ausgesendeten Leistung zu bestimmen.

5. Vorrichtung nach einem der vorhergehenden Ansprüche, wobei die Steuerung (23) ausgelegt, in Abhängigkeit von dem ausgegebenen Signal eine Aussendung von Hochfrequenz durch den Sender (24) zu unterbrechen.

6. Vorrichtung nach einem der vorhergehenden Ansprüche, wobei die Vorrichtung ausgelegt ist, über die Antenne eine Mehrzahl unterschiedlicher vorbestimmter Moden auszusenden, mittels einer Mehrzahl an Amplitudenmessern (25) eine Mehrzahl an Prüfamplituden zu erfassen, und in Abhängigkeit von den erfassten Prüfamplituden eine oder mehrere Prüfrelationen zu bestimmen.

7. Magnetresonanztomograph mit einer Vorrichtung nach einem der vorhergehenden Ansprüche, wobei der Sender (24) ausgelegt ist, einen Anregungspuls für Kernspins zu erzeugen.

8. Verfahren zum Erkennen einer Antennenspule mit nicht aktiver Verstimmungsvorrichtung, mit einer Vorrichtung nach einem der Ansprüche 1 bis 6, wobei das Verfahren die Schritte aufweist:
(S20) Ansteuern des Senders (24) durch die Steuerung (23), sodass der Sender (24) Hochfrequenzsignale mit unterschiedlichen vorbestimmten Amplitude über die Antenne aussendet;
(S30) Erfassen von Prüfamplituden mit dem Amplitudenmesser (24) in Abhängigkeit von den ausgesendeten Hochfrequenzsignalen;
(S40) Bestimmen einer Prüfrelation zwischen den vorbestimmten Amplituden und den erfassten Prüfamplituden;
(S50) Ausgeben eines Signals bei Abweichung der bestimmten Prüfrelation von einer vorbestimmten Referenzrelation, wobei die vorbestimmte Referenzrelation in der Steuerung (23) eine lineare Relation ist.

9. Verfahren nach Anspruch 8, wobei das Verfahren weiterhin den Schritt (S10) aufweist, eine Verstimmungsvorrichtung einer Antennenspule zu deaktivieren, wobei sich die Antennenspule in einem Bereich angeordnet ist, der in dem Schritt (S20) des Ansteuerns eine relevante Feldstärke der Hochfrequenzsignale aufweist.

10. Computerprogrammprodukt, welches direkt in einen Prozessor einer programmierbaren Steuerung (23) ladbar ist, mit Programmcode-Mitteln, um alle Schritte eines Verfahrens nach einem der Ansprüche 8 oder 9 auszuführen, wenn das Programmprodukt auf der Steuerung (23) ausgeführt wird.

11. Computerlesbares Speichermedium mit darauf gespeicherten elektronisch lesbaren Steuerinformationen, welche derart ausgestaltet sind, dass sie bei Verwendung des Speichermediums in einer Steuerung (23) eines Magnetresonanztomographen (1) nach Anspruch 7 das Verfahren nach einem der Ansprüche 8 oder 9 durchführen.

## Claims

1. Apparatus for detecting an antenna coil with a non-active detuning apparatus, wherein the apparatus has a transmitter (24), an antenna, an amplitude meter (25) and a controller (23),
wherein the controller (23) is designed to actuate the transmitter (24) such that it emits radio-frequency signals with different predetermined amplitudes via the antenna, to acquire testing amplitudes with the amplitude meter (25) as a function of the emitted signal, to determine a testing relationship between the predetermined amplitudes and the acquired testing amplitudes and, if the determined testing relationship deviates from a predetermined reference relationship, to emit a signal, wherein the predetermined reference relationship in the controller (23) is a linear relationship.

2. Apparatus according to claim 1, wherein the amplitude meter (25) has a directional coupler and is designed to acquire the testing amplitudes as a function of a reflected output.

3. Apparatus according to one of the preceding claims, wherein the controller (23) is designed to determine the testing relationship as a function of a phase of the testing amplitude acquired by the amplitude meter (25).

4. Apparatus according to one of the preceding claims, wherein the controller (23) is designed to determine the testing relationship as a function of an output emitted via the antenna.

5. Apparatus according to one of the preceding claims, wherein the controller (23) is designed to interrupt an emission of radio frequency by the transmitter (24) as a function of the output signal.

6. Apparatus according to one of the preceding claims, wherein the apparatus is designed to emit a plurality of different predetermined modes via the antenna, to acquire a plurality of testing amplitudes by means of a plurality of amplitude meters (25), and to determine one or more testing relationships as a function of the acquired testing amplitudes.

7. Magnetic resonance tomography system with an apparatus according to one of the preceding claims, wherein the transmitter (24) is designed to generate an excitation pulse for nuclear spins.

8. Method for detecting an antenna coil with a non-active detuning apparatus, with an apparatus according to one of claims 1 to 6, wherein the method has the steps:
(S20) Actuation of the transmitter (24) by the controller (23), so that the transmitter (24) emits radio-frequency signals with different predetermined amplitude via the antenna;
(S30) Acquisition of testing amplitudes with the amplitude meter (25) as a function of the emitted radio-frequency signals;
(S40) Determination of a testing relationship between the predetermined amplitudes and the acquired testing amplitudes;
(S50) Emission of a signal if the determined testing relationship deviates from a predetermined reference relationship, wherein the predetermined reference relationship in the controller (23) is a linear relationship.

9. Method according to claim 8, wherein the method furthermore has the step (S10) of deactivating a detuning apparatus of an antenna coil, wherein the antenna coil is arranged in a region which, in step (S20) of the actuation, has a relevant field strength of the radio-frequency signals.

10. Computer program product which is loadable directly into a processor of a programmable controller (23), having program code means in order to carry out all the steps of a method according to one of claims 8 or 9 when the program product is executed on the controller (23).

11. Computer-readable storage medium with electronically readable control information stored thereon, which is configured such that when using the storage medium in a controller (23) of a magnetic resonance tomography system (1) according to claim 7, it performs the method according to one of claims 8 or 9.

## Revendications

1. Dispositif de détection d'une bobine d'antenne à dispositif d'accord inactif, le dispositif comprenant un émetteur (24), une antenne, un mesureur (25) d'amplitude et une commande (23),
dans lequel la commande (23) est conçue pour commander l'émetteur (24) de manière à émettre, par l'antenne, des signaux de haute fréquence ayant des amplitudes différentes déterminées à l'avance, à détecter, par le mesureur (25) d'amplitude, des amplitudes de contrôle en fonction du signal émis, à déterminer une relation de contrôle entre les amplitudes déterminées à l'avance et les amplitudes de contrôles détectées et, s'il y a un écart entre la relation de contrôle déterminée et une relation de référence déterminée à l'avance, à émettre un signal, la relation de référence déterminée à l'avance étant une relation linéaire dans la commande (23).

2. Dispositif suivant la revendication 1, dans lequel le mesureur (25) d'amplitude a un coupleur directionnel et est conçu pour détecter les amplitudes de contrôle en fonction d'une puissance réfléchie.

3. Dispositif suivant l'une des revendications précédentes, dans lequel la commande (23) est conçue pour déterminer la relation de contrôle en fonction d'une phase de l'amplitude de contrôle détectée par le mesureur (25) d'amplitude.

4. Dispositif suivant l'une des revendications précédentes, dans lequel la commande (23) est conçue pour déterminer la relation de contrôle en fonction d'une puissance émise par l'antenne.

5. Dispositif suivant l'une des revendications précédentes, dans lequel la commande (23) est conçue pour, en fonction du signal émis, interrompre une émission de haute fréquence par l'émetteur (24).

6. Dispositif suivant l'une des revendications précédentes, dans lequel le dispositif est conçu pour émettre, par l'antenne, une pluralité de modes différents déterminés à l'avance, pour détecter, au moyen d'une pluralité de mesureurs (25) d'amplitude, une pluralité d'amplitudes de contrôle et pour déterminer, en fonction des amplitudes de contrôle détectées, une ou plusieurs relations de contrôle.

7. Tomographe à résonance magnétique ayant un dispositif suivant l'une des revendications précédentes, l'émetteur (24) étant conçu pour produire une impulsion d'excitation de spin nucléaire.

8. Procédé de détection d'une bobine d'antenne à dispositif d'accord inactif, comprenant un dispositif suivant l'une des revendications 1 à 6, le procédé ayant les stades :
(S20) commande de l'émetteur (20) par la commande (23), de manière à ce que l'émetteur (24) émette par l'antenne des signaux de haute fréquence d'amplitudes différentes déterminées à l'avance ;
(S30) détection d'amplitudes de contrôle par le mesureur (24) d'amplitudes en fonction des signaux de haute fréquence émis ;
(S40) détermination d'une relation de contrôle entre les amplitudes déterminées à l'avance et les amplitudes de contrôle détectées ;
(S50) émission d'un signal, s'il y a un écart entre la relation de contrôle déterminée et une relation de référence déterminée à l'avance, la relation de référence déterminée à l'avance étant une relation linéaire dans la commande (23).

9. Procédé suivant la revendication 8, dans lequel le procédé a, en outre, le stade (S10) de désactivation d'un dispositif d'accord d'une bobine d'antenne, la bobine d'antenne étant disposée dans une partie, qui, dans le stade (S20) de la commande, a une intensité de champ pertinente des signaux de haute fréquence.

10. Produit de programme d'ordinateur, qui peut être chargé directement dans un processeur d'une commande (23) programmable, comprenant des moyens de code de programme pour effectuer tous les stades d'un procédé suivant l'une des revendications 8 ou 9, lorsque le produit de programme est exécuté sur la commande (23).

11. Support de mémoire déchiffrable par ordinateur ayant des informations de commande déchiffrables électroniquement, qui y sont mises en mémoire et qui sont conformées de manière à effectuer, lors de l'utilisation du support de mémoire dans une commande (23) d'un tomographe (1) à résonance magnétique suivant la revendication 7, le procédé suivant l'une des revendications 8 ou 9.
